(19) Europäisches Patentamt European Patent Office Office européen des brevets

(11) **EP 1 909 806 B1**

(12) **EUROPEAN PATENT SPECIFICATION**

(45) Date of publication and mention of the grant of the patent:
**20.11.2013 Bulletin 2013/47**

(51) Int Cl.:
***A61K 36/00*** (2006.01)

(21) Application number: **05764200.1**

(22) Date of filing: **30.06.2005**

(86) International application number:
**PCT/US2005/023381**

(87) International publication number:
**WO 2006/007538 (19.01.2006 Gazette 2006/03)**

(54) **COMPOSITION AND METHOD FOR THE TREATMENT OF ASTHMA SYMPTOMS**

ZUSAMMENSETZUNG UND VERFAHREN ZUR BEHANDLUNG VON ASTHMASYMPTOMEN

COMPOSITION ET METHODE POUR LE TRAITEMENT DE SYMPTOMES ASTHMATIQUES

(84) Designated Contracting States:
**AT BE BG CH CY CZ DE DK EE ES FI FR GB GR HU IE IS IT LI LT LU MC NL PL PT RO SE SI SK TR**

(30) Priority: **01.07.2004 US 584929 P**

(43) Date of publication of application:
**16.04.2008 Bulletin 2008/16**

(60) Divisional application:
**12161057.0 / 2 491 936**

(73) Proprietor: **Azmazin Inc.**
**Michigan City, IN 46360 (US)**

(72) Inventor: **Baek, Sung H.**
**Snohomish, WA 98296 (US)**

(74) Representative: **Teall, Charlotte et al**
**Forresters**
**Skygarden**
**Erika-Mann-Strasse 11**
**80636 München (DE)**

(56) References cited:
| | |
|---|---|
| CN-A- 1 066 389 | CN-A- 1 088 819 |
| CN-A- 1 327 805 | CN-A- 1 395 947 |
| CN-A- 1 544 022 | CN-C- 1 059 327 |
| JP-A- 62 056 435 | KR-A- 2003 037 355 |
| KR-A- 20030 007 243 | KR-A- 20040 023 199 |
| KR-A- 20040 030 371 | US-A1- 2002 031 559 |
| US-B1- 6 524 627 | US-B1- 6 524 627 |

- **AKAMATSU H ET AL: "Effect of keigai-rengyo-to, a Japanese Kampo medicine, on neutrophil functions: A possible mechanism of action of keigai-rengyo-to in acne" JOURNAL OF INTERNATIONAL MEDICAL RESEARCH, CAMBRIDGE MEDICAL PUBLICATIONS LTD, GB, vol. 25, no. 5, 1 January 1997 (1997-01-01), pages 255-265, XP009125549 ISSN: 0300-0605**
- **BOWLER RUSSELL P: "Oxidative stress in the pathogenesis of asthma" CURRENT ALLERGY AND ASTHMA REPORTS, CURRENT SCIENCE, US, vol. 4, no. 2, 1 March 2004 (2004-03-01), pages 116-122, XP009125542 ISSN: 1529-7322**
- **TODA S. ET AL.: 'Effects of the Chinese Herbal Medicine 'Saiboku-to' on Histamine Release from and the Degranulation of Mouse Peritoneal Mast Cells Induced by Compound 48/80' JOURNAL OF ETHNOPHARMACOLOGY vol. 24, 1988, pages 303 - 309, XP002995192**

EP 1 909 806 B1

## Description

### Field of the Invention

The present invention relates generally to asthma treatment and more specifically it relates to compositions and methods for the treatment of asthma, which are efficacious for reducing and mitigating asthma symptoms in humans.

### Background Art

It can be appreciated that various asthma treatments have been in use for years. Typically, asthma treatments involve various inhaler devices and similar products, a number of "natural" asthma treatment formulas marketed in pill or capsule form, and various known drug products.

A primary problem with conventional asthma treatments is that such products are difficult to use effectively. Another problem with conventional asthma treatments are that such products are not effective, or are not adequate to significantly reduce asthma symptoms. Another problem with conventional asthma treatment compositions is that such products are awkward and cumbersome to use, such as the conventional products that deliver asthma medication by inhalation, thus compromising the treatment regimen compliance of the asthma patient.

While these treatments of the background art may be suitable for the particular purpose to which they marketed, they are not as suitable for effectively reducing and mitigating asthma symptoms in humans, particularly because such products can be difficult to use effectively. Also, many such products are awkward and cumbersome to use, such as the conventional "asthma inhaler."

In these respects, the herbal composition for the treatment of asthma symptoms according to the present invention substantially departs from the conventional concepts of the technologies of the background art, and in so doing provides a composition which has been primarily developed for the purpose of reducing and mitigating asthma symptoms in humans.

### Summary

In view of the foregoing disadvantages inherent in the known types of asthma treatments now present in the art, the present invention provides a new herbal composition comprising naturally sourced ingredients and which is easy to use, for the treatment of asthma symptoms, and methods wherein the same can be utilized for reducing and mitigating asthma symptoms in humans.

The general purpose of the present invention, which will be described subsequently in greater detail, is to provide new herbal compositions and methods for the treatment of asthma symptoms that have many of the advantages mentioned heretofore and many novel features that are not anticipated, rendered obvious, suggested, or even implied by any of the previously known asthma treatment compositions or methods, either alone or in any combination thereof.

To attain this, the present invention generally comprises the following extracted and concentrated herbal ingredients, in proportions effective to reduce or mitigate the symptoms commonly associated with asthma:

| | | |
|---|---|---|
| Platycodi Radix | Sileris Radix | Trichosanthis Radix |
| Poria Cocos | Forsythia Fructus | Atractylodis Alba Rhizoma |
| Ligustici Wallichii Rhizoma | Ginseng Radix | Fritillariae Bulbus |
| Auranti Fructus | Notoptergii Rhizoma et Radix | Scutellariae Radix |
| Bupleuri Radix | Angelicae Pubescentis Radix | Rhei Rhizoma et Radix |
| Peucedani Radix | Glycyrrhizae Radix | Citri Reticulatae Pericarpium |
| Menthe Herba | Zingiberis Recens Rhizoma | Massa Medicata Fermentata |
| Schizonepetae Herba | Angelicae Radix | |

Thus there has been outlined, rather broadly, the more important features of the invention in order that the detailed description thereof may be better understood, and in order that the present contribution to the art may be better appreciated. There are additional features of the invention that will be described hereinafter.

In this respect, before explaining at least one embodiment of the invention in detail, it is to be understood that the invention is not limited in its application to the details and to the arrangements of the components set forth in the following description. The invention is capable of other embodiments and of being practiced and carried out in various ways. Also, it is to be understood that the phraseology and terminology employed herein are for the sole purpose of the description of the present invention and should not be regarded as limiting.

A primary object of the present invention is to provide herbal compositions and methods for the treatment of asthma symptoms that will overcome the shortcomings of the background art as described above.

An object of the present invention is to provide herbal compositions and methods for the treatment of asthma symptoms, which are effective for reducing or mitigating asthma symptoms in humans.

Another obj ect is to provide herbal compositions for the treatment of asthma symptoms that are effective and simple and easy to use.

Another object is to provide herbal compositions and methods for their use, for the treatment of asthma symptoms, which are effective and encourage treatment regimen compliance in asthma patients.

Other objects and advantages of the present invention will become obvious to the reader and it is intended that these objects and advantages are also within the scope of the present invention as described herein.

To the accomplishment of the above and related objects, this invention may be embodied in the preferred forms described herein, attention being called to the fact, however, that such descriptions are illustrative only, and that changes may be made in the specific details thereof which are within the scope of the present invention.

Various other objects, features and attendant advantages of the present invention will become fully appreciated as the same becomes better understood when considered in conjunction with the accompanying graphical representations of the results of clinical research studies involving the novel compositions of the invention, in which like reference characters designate the same or similar parts throughout the several views, as follows:

**Description of the Preferred Embodiments**

The following describes a preferred embodiment of the herbal composition for the treatment of asthma symptoms in accordance with the present invention, which comprises the ingredients in the proportions of the total composition as set forth in the following table:

**Table 1**

| APPROXIMATE NUMBER OF PARTS (of total composition) | Name of Herb |
|---|---|
| 14 | Platycodi Radix |
| 10 | Poria Cocos |
| 10 | Ligustici Wallichii Rhizoma |
| 10 | Auranti Fructus |
| 10 | Bupleuri Radix |
| 10 | Peucedani Radix |
| 12 | Menthe Herba |
| 12 | Schizonepetae Herba |
| 10 | Sileris Radix |
| 10 | Forsythia Fructus |
| 8 | Ginseng Radix |
| 8 | Notoptergii Rhizoma et Radix |
| 5 | Angelicae Pubescentis Radix |
| 6 | Glycyrrhizae Radix |
| 10 | Zingiberis Recens Rhizoma |
| 5 | Angelicae Radix |
| 3 | Trichosanthis Radix |
| 3 | Atractylodis Alba Rhizoma |
| 2 | Fritillariae Bulbus |
| 2 | Scutellariae Radix |
| 1 | Rhei Rhizoma et Radix |

(continued)

| APPROXIMATE NUMBER OF PARTS (of total composition) | Name of Herb |
|---|---|
| 0.5 | Citri Reticulatae Pericarpium |
| 0.5 | Massa Medicata Fermentata |

It will be appreciated that the above parts of the total composition of each ingredient are not absolute, and that up to six of any of the foregoing ingredients in the preferred embodiment of the invention as set forth above can be replaced by other of the foregoing ingredients, or with inert ingredients, and the composition of the invention will still have a beneficial effect of reducing asthma symptoms. In accordance with the present invention it has been found that the percentage of each ingredient in the inventive composition can be varied up to about 33 percent, that is, increased or decreased by up to about 33 percent, and the composition will still be efficacious at reducing or mitigating asthma symptoms. Thus, since the above-listed parts of the composition provided by the invention do not add up to 100, it is to be appreciated that each ingredient is capable of being varied in amount in the composition such that it may be increased or decreased by up to about 33 percent in amount from the number of parts set forth in the above table, and that the overall resulting composition will still be efficacious in reducing or mitigating the symptoms of asthma. Most preferably, the percentage of such ingredients can each be increased or decreased up to about 10 percent in order to achieve the most preferred range of ingredients with the most effective reduction of asthma symptoms when the composition of the invention is used.

Studies were undertaken in order to test the effectiveness of the novel compositions provided by the present invention at reducing asthma symptoms in human subjects. The results are set forth in the following pages:

**Clinical Site:**

Bangor, Maine:

Marshall-Blum: Clinical Outcomes Specialists (parent company)

Herbal Research Clinic
Independent Medical Research Center
James M. Blum, Ph.D., CEO
Ronald I. Blum, M.D., Medical Director
**Official Institutional Review Board Project Title:** Herbal Support for Asthma: A Pilot, Prospective, Non-Randomized, Open-label Clinical Trial to Test for Efficacy and Short-Term Safety of Jiang Jing's Azmazin.

**Protocol**

**Design**

- A pilot, prospective, non-randomized, open-label, non-placebo-controlled clinical trial.
- This trial had Institutional Review Board (IRB) approval [due to Federal Drug Administration (FDA) regulations, details are only available upon request].
- This study was divided into three phases.
- In Phase 1, the duration was two (2) weeks in total; a single dose of the product was administered on day 1 followed by two weeks of measurements and observation.
- For Phase 2, all subjects who completed Phase 1 began an additional two (2) period; daily doses of the product were administered for 1 week accompanied by an additional 1 week of measurements and observation.
- For Phase 3, all subjects who completed Phase 2, were invited to take the product daily for one month. They returned to the clinic at the end of one-month for assessment.
- A blood draw was required at baseline, at 2, 4, and 8 weeks.
- Since this was an open-label trial, no randomization was performed.
- Subjects were recruited from the general population of the greater Bangor, Maine region; Subjects were chosen from those who answered radio ads for this trial.

- This clinical site is an independent research facility.
- A registered nurse or the principal investigator saw all subjects at each visit.
- Subjects were paid $100 upon completion of this trial through Phase 2;
  Subjects were paid an additional $25 upon completion of Phase 3.
- The major exclusion criteria included individuals with alcohol abuse and major health issues.

**Exclusion Criteria:**

- Subjects who are non-compliant with testing and treatment regimens.
- Subjects who express problems with ingredients in the active product.
- Subjects under the age of 18 or over the age of 70.
- Subjects with moderately severe co-morbid disease, the includes cardiac, pulmonary, renal, hepatic, or active cancer (determined in coordination with the study physician).

**Cautionary Criteria:**

- Subjects with any medical conditions
- Subjects who are taking any prescription medications, over-the-counter medications and/or any dietary supplements.

**Confounding Factors:**

- Age
- Gender
- Body, Mass Index (BMI)
- Current medicines
- Prior treatment for the condition and/or symptoms
- Alcohol consumption

**End-Points**

**Primary Outcomes:**

- Peak flow spirometry
- Frequency and Amount of asthma related medications
- Gross pulmonary functional status (subjective)
- Nursing assessment
- General health status
- Amount of daily energy
- P53 (blood test used as a marker for lung disease)

**Secondary Outcomes:**

- Blood pressure
- Pulse
- Respirations
- Selected portions of the SF-12 (Quality-of-Life)
- Overall well-being
- "Recommendation to Use" and "Continued Use of this Product"

**Analytical Methods**

**Peak Flow Analysis**

High/Low

Both the high and low for the 2-day baseline set was compared to the respective high and low for the end of Phase 1, Phase 2, and Phase 3.

Average

The average was calculated for each point of measurement (Baseline, Phase 1, Phase 2, and Phase 3) using all 8 reading for the two days (AM and PM)

Example:

|  |  |  |
|---|---|---|
| Baseline: | Day 1: | AM: 360 and 370 |
|  |  | PM: 340 and 330 |
|  | Day 2: | AM: 360 and 380 |
|  |  | PM: 340 and 340 |
| Phase 2: | Day 1: | AM: 470 and 480 |
|  |  | PM: 450 and 450 |
|  | Day 2: | AM: 480 and 490 |
|  |  | PM: 460 and 440 |

Baseline Low AM = 360
Baseline High AM = 380
Baseline Low PM = 330
Baseline High PM = 340
Baseline Average = (360 + 370 + 340 + 330 + 360 + 380 + 340 + 340)/8 = 352.5

Phase 2 Low AM = 470
Phase 2 High AM = 490
Phase 2 Low PM = 440
Phase 2 High PM = 460
Phase 2 Average = (470 + 480 + 450 + 450 + 480 + 490 + 460 + 440)8 = 465.0

Low AM Difference = 470 - 360 = 110
High AM Difference = 490 - 380 = 110
Low PM Difference = 440 - 330 = 110
High PM Difference = 460 - 340 = 120
Average Difference = 465.0 - 352.5 = 112.5
Percent Improvement = 112.5/352.5 = 31.9%

**Questionnaires:**

- Answers from the questionnaires were coded from 0 to 5, where 0 equals no symptoms and 5 equals feeling the worst one could be.
- Answers from the two time periods were subtracted from each subject to created the outcome measures [there are three different time periods that were compared to baseline: Phase 1-Baseline, Phsae 2-baseline, and Phase 3-Baseline]

*Example*

"How often can you climb two flights of stairs without experiencing any shortness of breath?"

| | |
|---|---|
| 0 | None of the time |
| 1 | Some of the time |
| 2 | Half the time |
| 3 | Most of the time |
| 4 | All of the time |
| 5 | Did Not Attempt |

*For example, a subject answering the question about stairs following Phase 2 and at Baseline give the following responses,*

| **Time** | **Response** |
|---|---|
| *Baseline* | *1* |
| *Phase 2* | *3* |

*The subtraction of the responses yields a point improvement for this subject on this question:*

$$3 - 1 = 2 \ point \ improvement$$

- These responses for the two time frames (baseline and end-of-Phase 2) for this question were summed. This forms the basis of the results.

**Percent Improvement:**

Percent improvement is calculated based on the baseline level, and is the difference divided b the baseline number.

From our example above:

$$[(3 - 1)/1] * 100 = 200\% \ improvement$$

**Categorical Analysis (Based on the Percent Improvement):**

- Three categories were made from the percent improvement distribution. The three groups are described below:

  ○ No Improvement: Less than 40% Improvement
  ○ Between 40 and 80%
  ○ 80% or Greater

The first group contains all those with negative responses (better scores at baseline) as well as a modest improvement. Individuals who did not experience a given symptom were assigned to the lowest group

**Statistical Significance**

These criteria were set prior to the analysis.

| | |
|---|---|
| Highly Significant: | $p < 0.05$ |
| Significant: | $p < 0.10$ |
| Statistical Trend: | $p < 0.15$ |

## Medications

The number and frequency of medications were assessed and rated retrospectively for each phase on a score from 0 to 9.

### Energy Levels:

Using the subject's subjective assessment of average energy, the energy score was created using the baseline level as the common parameter.

### Peak Flows:

The average peak flow for each subject was calculated using the methodology described earlier in this section.

### Nursing Assessment:

The nursing assessment was taken from the nursing notes in the subject's chart. This assessment was created during a thorough discussion between the subj ect and the nurse. From the point of view of a realistic assessment, this parameter may be the most important of all the summary variables.

### Functional Status:

The functional status is a combination of all the questions relating to the subject's functional ability. These included the following questions:

- How far can you walk before feeling short of breath?
- How often can you climb a flight of stairs without experiencing any shortness of breath?
- How often can you climb two flights of stairs without experiencing any shortness of breath?
- How often do you experience chest pain with any shortness of breath?
- How many times have you called or seen a healthcare provider for your asthma symptoms:
- How many asthma episodes have you experienced in an average week?
- How often has dust triggered wheezing?
- How often has smoking triggered wheezing?
- How many pillows have you used to sleep comfortably?
- How often do you wake up feeling short of breath?
- How often are you woken up by a feeling of shortness of breath?

The responses are recorded on a six-answer option.

## Clinical Trial Results

### Subject Numbers

**[0044]** Thirty-two (32) subjects were enrolled in this trial. Twenty-five subjects (25) started and completed Phase 1, twenty-three (23) completed Phase 2, and eleven (11) completed Phase 3.

One subject who completed just the first phase, and who was deemed sufficiently improved, stopped the study at this point. This subject should be included in numbers, who completed the study. Three subjects, who all completed Phase 2, were unable to participate in Phase 3 (2 because of minor discomforts and one because their job required them to travel). These three were subtracted from those eligible for Phase 3. Thus 12 subjects completed the study, out of 22 possible who started the study and were not eliminated (25 - 3 = 22).

### Baseline Characteristics

Since this is an open-label study, there are no between-group differences to assess. The baseline characteristics are listed in the section just prior to the outcome graphs.

|  | N (Out of 32) | Percent |
|---|---|---|
| Age (mean) | 37.3 | --- |
| Arthritis, rheumatoid | 3 | 9.4 |
| Asthma | 32 | 100 |
| Caffeine > 3 Cups/Day | 9 | 28.2 |
| Cancer (History of) | 4 | 12.5 |
| Cardiac | 0 | 0 |
| Cholesterol (elevated) | 7 | 21.9 |
| COPD | 2 | 6.3 |
| Diabetic | 1 | 3.1 |
| Depression | 8 | 25 |
| GI | 2 | 6.3 |
| Hypertension | 5 | 15.6 |
| Injury (Major) | 11 | 34.4 |
| Liver | 1 | 3.1 |
| Migraine | 9 | 28.1 |
| Renal | 0 | 0 |
| Smoker, Former | 9 | 29.1 |
| Smoker, Current | 5 | 16.1 |
| Major Surgery | 18 | 56.3 |
| Thyroid Disease | 2 | 6.3 |

Medical Risk Profile: One point awarded for each of the above medical conditions. This score does not include behavioral risks (smoking, caffeine, or alcohol use). This risk score is used to gauge a subject's medical health.

| Risk Score | Number of Subjects | Percent |
|---|---|---|
| 1 | 3 | 3.1 |
| 2 | 8 | 25.0 |
| 3 | 6 | 18.8 |
| 4 | 3 | 9.4 |
| 5 | 4 | 12.5 |
| 6 | 3 | 9.4 |
| 7 | 3 | 9.4 |
| 8 | 1 | 3.1 |
| 9 | 1 | 3.1 |
|  | 32 | 100 |

| | | |
|---|---|---|
| Low Risk: | 0 - 2 Points | 34.4% |
| Intermediate Risk: | 3 - 5 Points | 40.6% |
| High Risk | 6 + Points | 25.0% |

The risk profile of those seven subjects who were enrolled (signed a consent form) but did not start the study were not different than the twenty-five subjects who started the trial.

**Results:**

**Peak Flow Readings:**

|  | N | Minimum | Maximum | Mean | Std. Dev. |
|---|---|---|---|---|---|
| Baseline | 25 | 160 | 650 | 356.7 | 122 |
| Phase 1 (end) | 25 | 185 | 600 | 377.0 | 128 |
| Phase 2 (end) | 23 | 195 | 750 | 404.0 | 160 |
| Phase 3 (end) | 11 | 298 | 794 | 489.2 | 180 |

**Peak Flow: Differences from Baseline:**

| Time Frame | Differences | Percent Improvement Over Baseline |
|---|---|---|
| Baseline - Phase 1 | 20.3 liters/minute | 5.7% |
| Phase 1- Phase 2 | 27.0 l/m | 7.6% |
| Baseline - Phase 2 | 47.3 l/m | 13.3% |
| Phase 2 - Phase 3 | 85.2 l/m | 23.9% |
| Baseline - Phase 3 | 132.5 l/m | 37.1% |

**Nursing Assessment:**

0 = No improvement
10 = Maximum level of improvement

|  | N | Minimum | Maximum | Mean | Std. Dev. |
|---|---|---|---|---|---|
| Baseline | 25 | 0 | 0 | 0 | 0 |
| Phase 1 (end) | 25 | 0 | 8 | 2.0 | 1.8 |
| Phase 2 (end) | 23 | 0 | 7 | 3.7 | 1.9 |
| Phase 3 (end) | 11 | 0 | 9 | 6.3 | 2.3 |

**Medications Assessment:**

10 = Subject uses the most possible amount of asthma medication
0 = Subject uses no asthma medications

|  | N | Minimum | Maximum | Mean | Std. Dev. |
|---|---|---|---|---|---|
| Baseline | 25 | 0 | 10 | 5.1 | 2.7 |
| Phase 1 (end) | 25 | 0 | 9 | 4.5 | 2.6 |
| Phase 2 (end) | 23 | 0 | 9 | 4.1 | 2.5 |
| Phase 3 (end) | 11 | 0 | 8 | 4.0 | 2.1 |

**Energy Levels:**

|  | N | Minimum | Maximum | Mean | Std. Dev. |
|---|---|---|---|---|---|
| Baseline | 25 | 0 | 8 | 5.0 | 2.0 |
| Phase 1 (end) | 25 | 2 | 9 | 6.2 | 1.7 |
| Phase 2 (end) | 23 | 3 | 9 | 6.6 | 1.4 |
| Phase 3 (end) | 11 | 4 | 8 | 6.0 | 1.2 |

**Functional Levels: Differences from Baseline**

|  | N | Minimum | Maximum | Mean | Std. Dev. |
|---|---|---|---|---|---|
| Phase 1 (end) | 25 | -2.0 | 22 | 8.4 | 6.7 |
| Phase 2 (end) | 23 | -2.0 | 26 | 9.4 | 7.2 |
| Phase 3 (end) | 11 | -2.0 | 17 | 7.1 | 5.8 |

**Summary of the End-Points**

3+ = 'Exceptional Improvement'
2+ = 'Significant Improvement'
1+ = 'Some Improvement'
0 = No Improvement

| ID | Weeks Complete | 3+ | 2+ | 1+ | 0 | Risk Profile | Confounder |
|---|---|---|---|---|---|---|---|
| 1 | 2 | + |  |  |  | 9 | Finished the Study |
| 21 | 2 |  |  | + |  | 3 |  |
| 5 | 4 | + |  |  |  | 6 |  |
| 6 | 4 |  |  | + |  | 6 | Out-of-Town, Drop |
| 8 | 4 |  | + |  |  | 4 |  |
| 10 | 4 |  |  |  | + | 2 |  |
| 15 | 4 | + |  |  |  | 3 |  |
| 19 | 4 |  | + |  |  | 2 |  |
| 22 | 4 |  |  | + |  | 7 |  |

(continued)

| ID | Weeks Complete | 3+ | 2+ | 1+ | 0 | Risk Profile | Confounder |
|---|---|---|---|---|---|---|---|
| 23 | 4 | + | | | | 3 | |
| 26 | 4 | | + | | | 8 | Rash, Drop |
| 27 | 4 | + | | | | 3 | |
| 28 | 4 | | + | | | 1 | |
| 29 | 4 | | | + | | 7 | |
| 30 | 4 | | + | | | 2 | Itchy Eyes, Drop |
| 2 | 8 | + | | | | 5 | |
| 3 | 8 | + | | | | 7 | |
| 4 | 8 | + | | | | 3 | |
| 11 | 8 | + | | | | 3 | |
| 14 | 8 | + | | | | 2 | |
| 16 | 8 | | + | | | 6 | |
| 17 | 8 | | + | | | 4 | |
| 20 | 8 | | | + | | 1 | |
| 31 | 8 | + | | | | 5 | |
| 32 | 8 | + | | | | 4 | |
| Totals | N | 12 | 7 | 5 | 1 | | |
| | Percent | 48% | 28% | 20% | 4% | | |
| Results from a typical 'good' study | | 30% | 25% | 20% | 25% | | |
| Differences between 'Azmazin' and Other Good Products | | 18% | 3% | 0 | 21% | | |

For the summary assessment, forty-eight percent (12/25) of the subjects were rated a 3+ level of improvement, considered dramatic. Twenty-eight percent (7/25) were rated 2+, while twenty percent (5/25) rated 1+, and four percent (1/25) did not have any improvement.

**Physiological Parameters:**

**Differences from baseline at End-of-Study:**

| Parameter | Mean Difference | Lower 95% C.I. | Upper 95% C.I. |
|---|---|---|---|
| **Systolic BP** | 2.4 torr | -4.06 | 8.86 |
| **Diastolic BP** | -0.133 torr | -7.20 | 6.40 |
| **Pulse** | -0.80 / minute | -7.53 | 5.93 |
| **Respiration** | -1.0 / minute | -2.55 | 0.55 |
| **Weight** | 0.16 pounds | -3.21 | 3.53 |
| **C.I. = Confidence Intervals** | | | |

**Continue-to-Use and Recommend the Product**

Seventy-eight percent (18/23) of the subject's at their last clinic visit reported that they would continue to use the product while an additional nine percent (2/23) were uncertain. Thirteen percent (3/23) reported that they would not continue-to-use the product. Two subjects failed to complete this field.

Seventy-nine percent (19/24) of the subjects at their last clinic visit reported that they would recommend the product while an additional seventeen percent (4/24) were uncertain. Four percent (1/24) reported that they would not recommend the product. One subject failed to complete this field.

## Adverse Event Reports

There were no serious adverse events reported in this trial. There were two subjects who reported minor complaints during Phase 2 of the trial. One reported itchy eyes and another reported a rash. These episodes were brief in nature but the subjects decided to withdraw from the study.

There were no clinical differences across the different time frames for systolic and diastolic blood pressure, pulse, and respirations, indicating that these physiological parameters were unchanged by the product, despite the improvements in the various end-points.

## Conclusions

The efficacy of the product "Azmazin" which had been formulated in accordance with the present invention appears to be very strong with respect to the treatment of symptoms of asthma based on the three phases of this clinical trail. All of the major symptoms associated with asthma of the subjects were improved while  on the product, and many showed a dose-dependent relationship. The increase was approximately equal for Phase 1 and 2 (at 20.3 and 27.0 point increase respectively), but increased dramatically at the end of Phase 3 (one month on product).

Excluding the peak flow changes, the nursing assessment is the most important parameter in this study because it involves a professional assessment. The baseline nursing level was purposely set at 0. It increased to 2.0 at the end of Phase 1, to 3.7 at the end of Phase 2, and rose dramatically to 6.3 at the end of Phase 3. The three-fold increase from Phase 1 to Phase 3 shows a linear pattern of improvement consistent with a dose-response relationship.

The baseline level of medications was just over 5 and dropped slightly over the course of the study to end at 4.0. Although there were dramatic improvements in peak flow and nursing assessments, the medication improvement was not too large. There may be several reasons for these results. First, subjects were told not to change their base medications unless they discussed possible modifications with their healthcare provider. Secondly, several subjects had colds and seasonal exacerbations during the trial requiring more rescue inhaler use than at the baseline period.

The energy levels increased from 5 to a peak of 6.6 at the end of Phase 2 and dropped down to 6.0 at the end of Phase 3. Similarly, the functional summary parameter increased 8 points over baseline at Phase 1 and then to 9.4 points at Phase 2. There was a slight drop off to 7 at Phase 3. The question is why both of these parameters demonstrated a drop off at the end. It is suspected that individuals tend to forget their baseline levels under conditions of chronic disease and change their expectations during treatment. As an example, an individual with chronic pain might express a level of pain at 8 (out of 10 points) prior to treatment. As this person heals, their expectations change. After several weeks, they forget that they started at an 8 and mark down only a 6, even though they have regained a great deal of relief. This scenario of changing expectation is well described in the medical literature.

The overall rating of the five end-points showed that almost half of the subjects rated the top level (3+) and an additional 28% scored a 2+ rating. Only 4% rated no improvement. While this rating was subjective, it was based on a combination of subjective (nursing, energy, and functional data) and objective data (peak flow, medications). The nursing assessment was crucial to this summary parameter as it represents a professional assessment. The overall impression of these various indicators is that the product worked exceptionally well at reducing chronic  asthma symptoms and allowing subjects to recover their functional abilities.

As one examines the individual set of graphs for all 25 subjects, there are a number of inconsistencies that commonly occur in human clinical trials. There are many factors responsible for these bumps in the data. One of the primary causes is that asthma is a chronic condition that tends to have highs and lows regardless of treatment. As discussed previously, changing expectations during the healing process may be one of the causes of this phenomenon. It is believed that peak flow reading should be supervised by a nurse to reduce variability.

There appear to be no drawbacks to this product with respect to safety as determined by this pilot trial.

The strengths of this trial include a moderately large sample size, inclusionary criteria that allowed a wide range of medical conditions (baseline characteristics) indicative of a normal, relatively healthy adult population, combination of subjective and objective outcomes, and a protocol standardization that reflects a typical asthma patient. As this study has been assessed by a board-certified pulmonologist, he was duly impressed by these preliminary results and has suggested that with restriction of inclusionary criteria the results may be even more impressive.

The limitations of this study include the lack of a placebo, lack of racial diversification, short duration of taking the product, lack of Forced Expiratory Volume in One Second (FEV1) measurements, and the inclusion of individuals over 50 as well as other criteria that may have diluted the results.

It will be appreciated that the ingredients of the novel compositions provided by the invention are commercially available from well-known vendors of herbal substances. Examples of such vendors carrying one or more of the ingredients described herein in their catalogs or inventories for commercial sale are:

1. Herbalcom
4124 Browns Valley Road, Napa, CA, 94558, 1-877-818-4115
2. Ancient Way Herbs
905 Main Street, #409, Klamath Falls, OR, 97601. 1-541-884-6377
3. Herb Pharm
20260 Williams Hwy.,Williams OR, 97544, 1-541-846-6262
4. San Francisco Herb
47444 Kato Road, Fremont, CA 94538, 1-415-861-7174
5. Starwest Botanicals
11253 Trade Center Drive, Rancho Cardova, CA 95742, 1-888273-4372
6. Frontier Natural Products
3021 78th St., Norway, IA 52318, 1-800-669-3275

Each ingredient in the compositions provided by the invention, in being produced prior to being formulated into the compositions of the invention, are extracted from raw herbal products using conventional, well known extraction processes, and concentrated, using conventional procedures well known in the art, from three to four times before being introduced to the composition and mixed with the other ingredients of the compositions as described herein. Accordingly, there can be expected to be up to about a 33 percent variation in the amount of each original raw herbal ingredient when it is introduced into a particular composition. Descriptions of the major raw herbal ingredients and their published and known properties are set forth in the following pages:

In use, the composition provided by the invention is utilized as described in the following pages:

**Dosing:**

**Phase 1:**

- Six capsules between meals with water
- This was a one-time event; capsules were taken in the clinic under observation by the nurse or principal investigator

**Phase 2:**

- Dosing: Daily doses were administered for the first week (7 days for a total of 42 capsules)
- Doses were administered at approximately the same time each day
- Dose: Six (6) capsules between meals with water

**Phase 3:**

- Dosing: Daily doses were administered for the entire month (approx 30 days for a total of 180 capsules)
- Doses were administered at approximately the same time each day
- Dose: Six (6)) capsules between meals with water

**Inclusion Criteria:**

- Men or women with diagnosed and under-controlled asthma and reporting an interest in taking part in a trial assessing the product;
- Age range: 18 - 70;
- Subjects who pass a compliance screening test;
- Subjects who believe they are able to tolerate the active product;
- Subjects who sign a consent form.

## EP 1 909 806 B1

Note: Under-controlled asthma is defined as those individuals requiring rescue inhalation of at least two times a week.

Thus, it is to be appreciated that a novel method for the treatment of asthma symptoms in a patient, which method comprises the steps of treating the patient with an effective amount of the composition of the invention as herein described, is also provided in accordance with the teachings of the present invention.

As to a further discussion of the methods provided by the invention and the manner of usage of the invention, the same should be apparent to those skilled in the art from the above description. Accordingly, no further discussion relating to the manner of usage will be provided.

With respect to the above description then, it is to be realized that the optimum composition of the invention, including variations thereof that are possible without departing from the usefulness thereof, are deemed readily apparent and obvious to one skilled in the art, and all equivalent relationships to those described in the specification are intended to be encompassed by the present invention.

## Claims

1. A composition for use in treating symptoms of asthma, consisting of a mixture of ingredients effective to reduce the symptoms of asthma in humans, said ingredients consisting of Platycodi Radix, Poria Cocos, Ligustici Wallichii Rhizoma, Auranti Fructus, Bupleuri Radix, Peucedani Radix, Menthe Herba, Schizonepetae Herba, Sileris Radix, Forsythia Fructus, Ginseng Radix, Notoptergii Rhizoma et Radix, Angelicae Pubescentis Radix, Glycyrrhizae Radix, Zingiberis Recens Rhizoma, Angelicae Radix, Trichosanthis Radix, Atractylodis Alba Rhizoma, Fritillariae Bulbus, Scutellariae Radix, Rhei Rhizoma et Radix, Citri Reticulatae Pericarpium and Massa Medicata Fermentata.

2. The composition for the use as claimed in Claim 1 wherein said ingredients are pretreated by a means selected from a group consisting of extraction, concentration, and combinations thereof.

3. The composition for the use as claimed in Claim 2 wherein said each extracted and concentrated ingredient is present in parts of the total composition, consisting of 15 parts Platycodi Radix, 10 parts Poria Cocos, 10 parts Ligustici Wallichii Rhizoma, 10 parts Auranti Fructus, 10 parts Bupleuri Radix, 10 parts Peucedani Radix, 12 parts Menthe Herba, 12 parts Schizonepetae Herba, 10 parts Sileris Radix, 10 parts Forsythia Fructus, 8 parts Ginseng Radix, 8 parts Notoptergii Rhizoma et Radix, 5 parts Angelicae Pubescentis Radix, 6 parts Glycyrrhizae Radix, 10 parts Zingiberis Recens Rhizoma, 5 parts Angelicae Radix, 3 parts Trichosanthis Radiz, 3 parts Atractylodis Alba Rhizoma, 2 parts Fritillariae Bulbus, 2 parts Scutellariae Radix, 1 part Rhei Rhizoma et Radix, 0.5 Citri Reticulatae Pericarpium, 0.5 parts Massa Medicata Fermentata, and combinations thereof, wherein the percentage of each extracted and concentrated ingredient can be increased or decreased by up to 33% in amount from the number of parts set forth above.

4. A composition of claims 1, 2 or 3 for use in the treatment of asthma symptoms in a patient, which method comprises the steps of treating the patient with an effective amount of the composition.

5. A composition consisting of Platycodi Radix, Poria Cocos, Ligustici Wallichii Rhizoma, Auranti Fructus, Bupleuri Radix, Peucedani Radix, Menthe Herba, Schizonepetae Herba, Sileris Radix, Forsythia Fructus, Ginseng Radix, Notoptergii Rhizoma et Radix, Angelicae Pubescentis Radix, Glycyrrhizae Radix, Zingiberis Recens Rhizoma, Angelicae Radix, Trichosanthis Radix, Atractylodis Alba Rhizoma, Fritillariae Bulbus, Scutellariae Radix, Rhei Rhizoma et Radix, Citri Reticulatae Percarpium and Massa Medicata Fermentata.

6. The composition of claim 5 wherein each ingredient is present in parts of the total composition, consisting of 15 parts Platycodi Radix, 10 parts Poria Cocos, 10 parts Ligustici Wallichii Rhizoma, 10 parts Auranti Fructus, 10 parts Bupleuri Radix, 10 parts Peucedani Radix, 12 parts Menthe Herba, 12 parts Schizonepetae Herba, 10 parts Sileris Radix, 10 parts Forsythia Fructus, 8 parts Ginseng Radix, 8 parts Notoptergii Rhizoma et Radix, 5 parts Angelicae Pubescentis Radix, 6 parts Glycyrrhizae Radix, 10 parts Zingiberis Recens Rhizoma, 5 parts Angelicae Radix, 3 parts Trichosanthis Radiz, 3 parts Atractylodis Alba Rhizoma, 2 parts Fritillariae Bulbus, 2 parts Scutellariae Radix, 1 part Rhei Rhizoma et Radix, 0.5 Citri Reticulatae Pericarpium, 0.5 parts Massa Medicata Fermentata, and combinations thereof, wherein the percentage of each ingredient can be increased or decreased by up to 33% in amount from the number of parts set forth above.

**EP 1 909 806 B1**

**Patentansprüche**

1. Zusammensetzung zur Verwendung bei der Behandlung von Symptomen von Asthma, die aus einer Mischung von Bestandteilen besteht, die zur Verringerung der Symptome von Asthma in Menschen wirksam ist, wobei die Bestandteile aus Platycodi radix, Poria cocos, Ligustici wallichii rhizoma, Auranti fructus, Bupleuri radix, Peucedani radix, Menthae herba, Schizonepetae herba, Sileris radix, Forsythiae fructus, Ginseng radix, Notopterygii rhizoma et radix, Angelicae pubescentis radix, Glycyrrhizae radix, Zingiberis recens rhizoma, Angelicae radix, Trichosanthis radix, Atractylodis alba rhizoma, Fritillariae bulbus, Scutellariae radix, Rhei rhizoma et radix, Citri reticulatae pericarpium und Massa medicata fermentata bestehen.

2. Zusammensetzung zur Verwendung nach Anspruch 1, wobei die Bestandteile durch ein Mittel vorbehandelt werden, das aus einer Gruppe bestehend aus Extraktion, Konzentration und Kombinationen davon ausgewählt ist.

3. Zusammensetzung zur Verwendung nach Anspruch 2, wobei jeder extrahierte und konzentrierte Bestandteil in Teilen der Gesamtzusammensetzung vorliegt, die aus 15 Teilen Platycodi radix, 10 Teilen Poria cocos, 10 Teilen Ligustici wallichii rhizoma, 10 Teilen Auranti fructus, 10 Teilen Bupleuri radix, 10 Teilen Peucedani radix, 12 Teilen Menthae herba, 12 Teilen Schizonepetae herba, 10 Teilen Sileris radix, 10 Teilen Forsythiae fructus, 8 Teilen Ginseng radix, 8 Teilen Notopterygii rhizoma et radix, 5 Teilen Angelicae pubescentis radix, 6 Teilen Glycyrrhizae radix, 10 Teilen Zingiberis recens rhizoma, 5 Teilen Angelicae radix, 3 Teilen Trichosanthis radix, 3 Teilen Atractylodis alba rhizoma, 2 Teilen Fritillariae bulbus, 2 Teilen Scutellariae radix, 1 Teil Rhei rhizoma et radix, 0,5 Teilen Citri reticulatae pericarpium, 0,5 Teilen Massa medicata fermentata und Kombinationen davon besteht, wobei der Prozentanteil jedes extrahierten und konzentrierten Bestandteils von der oben dargelegten Zahl der Teile um bis zu 33 % in Bezug auf die Menge erhöht oder gesenkt werden kann.

4. Zusammensetzung nach Anspruch 1, 2 oder 3 zur Verwendung bei der Behandlung von Asthmasymptomen in einem Patienten, wobei das Verfahren die Schritte des Behandelns des Patienten mit einer wirksamen Menge der Zusammensetzung umfasst.

5. Zusammensetzung, die aus Platycodi radix, Poria cocos, Ligustici wallichii rhizoma, Auranti fructus, Bupleuri radix, Peucedani radix, Menthae herba, Schizonepetae herba, Sileris radix, Forsythiae fructus, Ginseng radix, Notopterygii rhizoma et radix, Angelicae pubescentis radix, Glycyrrhizae radix, Zingiberis recens rhizoma, Angelicae radix, Trichosanthis radix, Atractylodis alba rhizoma, Fritillariae bulbus, Scutellariae radix, Rhei rhizoma et radix, Citri reticulatae pericarpium und Massa medicata fermentata besteht.

6. Zusammensetzung nach Anspruch 5, wobei jeder Bestandteil in Teilen der Gesamtzusammensetzung vorliegt, die aus 15 Teilen Platycodi radix, 10 Teilen Poria cocos, 10 Teilen Ligustici Wallichii rhizoma, 10 Teilen Auranti fructus, 10 Teilen Bupleuri radix, 10 Teilen Peucedani radix, 12 Teilen Menthae herba, 12 Teilen Schizonepetae herba, 10 Teilen Sileris radix, 10 Teilen Forsythiae fructus, 8 Teilen Ginseng radix, 8 Teilen Notopterygii rhizoma et radix, 5 Teilen Angelicae pubescentis radix, 6 Teilen Glycyrrhizae radix, 10 Teilen Zingiberis recens rhizoma, 5 Teilen Angelicae radix, 3 Teilen Trichosanthis radix, 3 Teilen Atractylodis alba rhizoma, 2 Teilen Fritillariae bulbus, 2 Teilen Scutellariae radix, 1 Teil Rhei rhizoma et radix, 0,5 Teilen Citri reticulatae pericarpium, 0,5 Teilen Massa medicata fermentata und Kombinationen davon besteht, wobei der Prozentanteil jedes Bestandteils von der oben dargelegten Zahl der Teile um bis zu 33 % in Bezug auf die Menge erhöht oder gesenkt werden kann.

**Revendications**

1. Composition destinée à être utilisée dans le traitement de symptômes asthmatiques, consistant en un mélange d'ingrédients permettant de réduire des symptômes asthmatiques chez l'homme, lesdits ingrédients consistant en Radix Platycodi, Poria Cocos, Rhizoma Ligustici Wallichii, Fructus Auranti, Radix Bupleuri, Radix Peucedani, Herba Menthae, Herba Schizonepetae, Radix Sileris, Fructus Forsythia, Radix Ginseng, Radix et Rhizoma Notoptergii, Radix Angelicae Pubescentis, Radix Glycyrrhizae, Rhizoma Zingiberis Recens, Radix Angelicae, Radix Trichosanthis, Rhizoma Atractylodis Alba, Bulbus Fritillariae, Radix Scutellariae, Radix et Rhizoma Rhei, Pericarpium Citri Reticulatae et Massa Medicata Fermentata.

2. Composition destinée à l'utilisation selon la revendication 1, dans laquelle lesdits ingrédients sont prétraités à l'aide d'un moyen choisi dans le groupe constant en une extraction, une concentration et leurs combinaisons.

**3.** Composition destinée à l'utilisation selon la revendication 2, dans laquelle chacun desdits ingrédients extraits et concentrés est présent en parties de la composition totale, consistant en 15 parties de Radix Platycodi, 10 parties de Poria Cocos, 10 parties de Rhizoma Ligustici Wallichii, 10 parties de Fructus Auranti, 10 parties de Radix Bupleuri, 10 parties de Radix Peucedani, 12 parties de Herba Menthae, 12 parties de Herba Schizonepetae, 10 parties de Radix Sileris, 10 parties de Fructus Forsythia, 8 parties de Radix Ginseng, 8 parties de Radix et Rhizoma Notoptergii, 5 parties de Radix Angelicae Pubescentis, 6 parties de Radix Glycyrrhizae, 10 parties de Rhizoma Zingiberis Recens, 5 parties de Radix Angelicae, 3 parties de Radix Trichosanthis, 3 parties de Rhizoma Atractylodis Alba, 2 parties de Bulbus Fritillariae, 2 parties de Radix Scutellariae, 1 partie de Radix et Rhizoma Rhei, 0,5 partie de Pericarpium Citri Reticulatae, 0,5 partie de Massa Medicata Fermentata, et leurs combinaisons, le pourcentage de chacun des ingrédients extraits et concentrés pouvant être augmenté ou diminuer au maximum de 33 % en quantité par rapport au nombre de parties indiqués précédemment.

**4.** Composition selon la revendication 1, 2 ou 3, destinée à être utilisée dans le traitement de symptômes asthmatiques chez un patient, le procédé comprenant l'étape consistant à traiter le patient avec une quantité acceptable de la composition.

**5.** Composition consistant en Radix Platycodi, Poria Cocos, Rhizoma Ligustici Wallichii, Fructus Auranti, Radix Bupleuri, Radix Peucedani, Herba Menthae, Herba Schizonepetae, Radix Sileris, Fructus Forsythia, Radix Ginseng, Radix et Rhizoma Notoptergii, Radix Angelicae Pubescentis, Radix Glycyrrhizae, Rhizoma Zingiberis Recens, Radix Angelicae, Radix Trichosanthis, Rhizoma Atractylodis Alba, Bulbus Fritillariae, Radix Scutellariae, Radix et Rhizoma Rhei, Pericarpium Citri Reticulatae et Massa Medicata Fermentata.

**6.** Composition selon la revendication 5, dans laquelle chaque ingrédient est présent en parties de la composition totale, consistant en 15 parties de Radix Platycodi, 10 parties de Poria Cocos, 10 parties de Rhizoma Ligustici Wallichii, 10 parties de Fructus Auranti, 10 parties de Radix Bupleuri, 10 parties de Radix Peucedani, 12 parties de Herba Menthae, 12 parties de Herba Schizonepetae, 10 parties de Radix Sileris, 10 parties de Fructus Forsythia, 8 parties de Radix Ginseng, 8 parties de Radix et Rhizoma Notoptergii, 5 parties de Radix Angelicae Pubescentis, 6 parties de Radix Glycyrrhizae, 10 parties de Rhizoma Zingiberis Recens, 5 parties de Radix Angelicae, 3 parties de Radix Trichosanthis, 3 parties de Rhizoma Atractylodis Alba, 2 parties de Bulbus Fritillariae, 2 parties de Radix Scutellariae, 1 partie de Radix et Rhizoma Rhei, 0,5 partie de Pericarpium Citri Reticulatae, 0,5 partie de Massa Medicata Fermentata, et leurs combinaisons, le pourcentage de chaque ingrédient pouvant être augmenté ou diminué au maximum de 33 % en quantité par rapport au nombre de parties indiqués précédemment.